(19) Europäisches Patentamt
European Patent Office
Office européen des brevets

(11) Veröffentlichungsnummer: **0 363 836 A2**

# (12) EUROPÄISCHE PATENTANMELDUNG

(21) Anmeldenummer: **89118566.2**

(51) Int. Cl.[5]: **A61K 31/55**

(22) Anmeldetag: **06.10.89**

Patentansprüche für folgende Vertragsstaaten: ES + GR.

(30) Priorität: **11.10.88 DE 3834532**

(43) Veröffentlichungstag der Anmeldung: **18.04.90 Patentblatt 90/16**

(84) Benannte Vertragsstaaten: **AT BE CH DE ES FR GB GR IT LI LU NL SE**

(71) Anmelder: **BEHRINGWERKE Aktiengesellschaft**
**Postfach 1140**
**D-3550 Marburg 1(DE)**

(72) Erfinder: **Schorlemmer, Hans-Ulrich, Dr.**
**Am Kirschenwald 2**
**D-3550 Marburg(DE)**
Erfinder: **Sedlacek, Hans Harald, Dr.**
**Sonnenhang 3**
**D-3550 Marburg(DE)**

(74) Vertreter: **Klein, Otto, Dr. et al**
**Hoechst AG Zentrale Patentabteilung**
**Postfach 80 03 20**
**D-6230 Frankfurt am Main 80(DE)**

## (54) Verwendung von Homopiperazinen zur Herstellung eines Arzneimittels für das Vorbeugen gegen und die Behandlung von Organabstossungsreaktionen.

(57) Es wird beschrieben, daß Verbindungen der Formel I

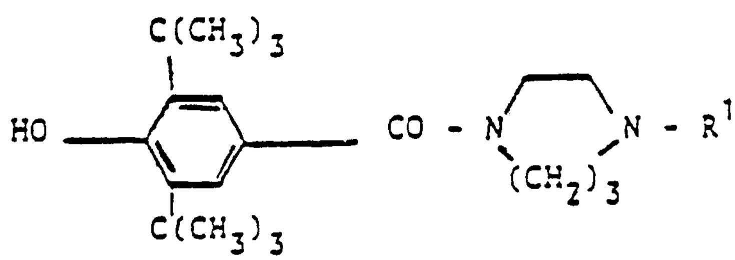

in der $R^1$ ein Wasserstoffatom, eine gradkettige oder verzweigte ($C_1$-$C_6$)-Alkoxycarbonyl-gruppe, vorzugsweise $CH_3OCO$, $C_2H_5OCO$ oder tert.-$C_{4-9}OCO$, oder die Benzyloxycarbonylgruppe, die im Phenylteil substituiert sein kann, bedeutet, und ihre physiologisch verträglichen Salze zur Herstellung eines Arzneimittels für Mensch und Tier für das Vorbeugen und die Behandlung von Organabstoßungsreaktionen verwendet werden können.

EP 0 363 836 A2

# Verwendung von Homopiperazinen zur Herstellung eines Arzneimittels für das Vorbeugen gegen und die Behandlung von Organabstoßungsreaktionen

Die Erfindung betrifft die Verwendung einer Verbindung der Formel I

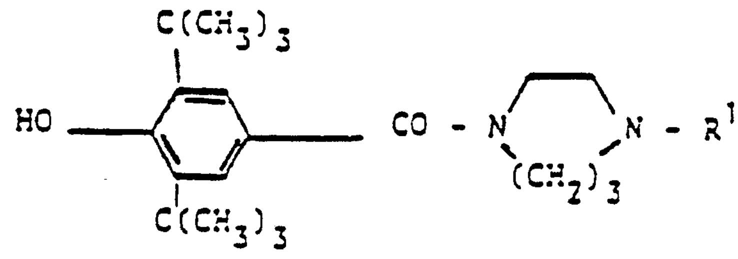

in der $R^1$ ein Wasserstoffatom, eine gradkettige oder verzweigte ($C_1$-$C_6$) -Alkoxycarbonyl-gruppe, vorzugsweise $CH_3OCO$, $C_2H_5OCO$ oder tert.-$C_4H_9OCO$, oder die Benzyloxycarbonyl-gruppe, die im Phenylteil substituiert sein kann, bedeutet, und ihre physiologisch verträglichen Salze zur Herstellung eines Arzneimittels für Mensch und Tier für das Vorbeugen und die Behandlung von Organabstoßungsreaktionen.

Diese Verbindungen sowie Verfahren zu ihrer Herstellung sowie ihre Verwendung für die Behandlung von entzündlichen Erkrankungen, insbesondere rheumatischen Erkrankungen, sind in der veröffentlichten deutschen Patentanmeldung P 37 02 755 beschrieben.

Nach der Transplantation von körperfremdem Gewebe kommt es zu einer immunologischen Reaktion gegen das als fremd erkannte transplantierte Organ, was zu einer Abstoßung führt. Es ist daher notwendig, das Immunsystem des Organempfängers zu unterdrücken, um das Überleben des Transplantates zu gewährleisten.

Es wurde nun überraschend gefunden, daß die Verbindungen der Formel I neben ihrer antientzündlichen und antirheumatischen Wirkung bei warmblütigen Säugern in einer Dosis, welche optimal zwischen 1 und 20 mg/kg Körpergewicht liegt und oral oder parenteral ein- bis mehrfach verabreicht wird, das Überleben transplantierter Organe verlängern, ohne dabei toxische Nebenwirkungen zu zeigen. Damit sind diese Verbindungen als Therapeutika besonders bei Transplantationen geeignet. Im Tierversuch ist es beispielsweise möglich, ein Hauttransplantat auf Fischer-Ratten durch die Gabe von den oben genannten Präparaten so günstig zu beeinflussen, daß es etwa 30 Tage überlebt, während in der Kontrollgruppe alle Hauttransplantate am Tag 17 abgestoßen sind.

Gegenstand der Erfindung ist demzufolge die Verwendung einer Verbindung der Formel I zur Herstellung eines Arzneimittels für das Vorbeugen und die Behandlung von Organabstoßungsreaktionen.

Die wirksame therapeutische Menge liegt vorzugsweise, jedoch nicht ausschließlich, im Bereich von 1-50 mg/kg Körpergewicht, sowohl bei parenteraler (ip) als auch bei oraler (po) Verabreichung.

Die Wirkstoffe können alleine gegeben oder auch mit anderen Arzneimitteln kombiniert werden, die Transplantationen günstig beeinflussen. Für die parenterale, speziell intravenöse Verabreichung kommen Lösungen oder Suspensionen der Wirkstoffe in einem pharmazeutisch verträglichen Vektor in Betracht, vorzugsweise Pflanzenöl wie Erdnußöl oder Sesamöl sowie alkoholische Lösungen z.B. Äthanol, Propandiol oder Glycerin oder in Gemischen der vorgenannten Lösungsmittel. Zur Herstellung wäßriger Lösungen werden die Wirkstoffe vorzugsweise in Form wasserlöslicher, physiologisch verträglicher Salze eingesetzt. Die Zubereitungen können die üblichen Hilf-und Trägerstoffe enthalten. Als solche kommen beispielsweise Füllstoffe, Emulgatoren, Gleit- und Pufferstoffe und geschmackskorrigierende Agenzien in Frage.

Die entsprechend der Erfindung verwendeten Verbindungen besitzen in den hierfür wirksamen Konzentrationsbereichen keine Toxizität und führen nicht zur lokalen Granulombildung.

Im Folgenden wird die Wirkung der Substanzen in Standardtestmethoden beispielhaft erläutert. Die herangezogenen Testmodelle sind bekanntermaßen für die Beurteilung von Therapeutika und deren Wirkungsqualität bei Transplantationen besonders gut geeignet.

Beispiel:

Einfluß von Homopiperazinen auf die Überlebenszeit von transplantierter Rattenschwanzhaut.

In einem Experimentalansatz wurden Schwanzhautstückchen von Lewis Ratten mit einer Größe von etwa 0.5 x 1.0 cm auf den Schwanz von Fischer Ratten verpflanzt. In einem weiteren Modell wurde die Schwanzhaut von DA Ratten auf Lewis Ratten transplantiert. Die transplantierten Hautstücke werden vom Immunsystem der Empfängertiere als fremd erkannt und abgestoßen. Wie in Tabelle I zu sehen ist, betrug die Transplantüberlebenszeit im Lewis-Fischer-Rattenmodell in den Kontrollgruppen, die nur mit dem Lösungsmittel behandelt wurden zwischen 16 und 18 Tagen. Die Verbindung der Formel I mit $R_1$ = H wurde in verschiedenen Konzentrationen (1, 5, 10, 20 und 40 mg/kg Körpergewicht pro Injektion) an 10 aufeinanderfolgenden Tagen, beginnend mit dem Tag der Transplantation, entweder oral oder intraperitoneal verabreicht. Hierbei zeigt sich, daß sich durch verschiedene Konzentrationen der Substanz die Überlebenszeiten der Transplantate dosisabhängig von 15.8 ± 2.4 auf 26.8 ± 1.3 bzw. 18.4 ± 1.5 auf 28.4 ± 1.1 erhöhen ließen, wobei 10 mg/kg eine optimal wirksame Dosis zu sein scheinen.

Auch im anderen Transplantationsmodell mit DA Ratten (Spender) und Lewis Ratten (Empfänger) kommt es zu einer Verlängerung der Transplantatüberlebenszeiten durch die Gabe von Homopiperazinen. Hier stoßen die nur mit dem Lösungsmittel behandelten Kontrolltiere das Transplantat ca. am Tag 12 ab. Durch die Behandlung (p.o. oder i.p. Applikation) mit unterschiedlichen Konzentrationen von Homopiperazinen (5-50 mg/kg Körpergewicht pro Injektion) an 5 aufeinanderfolgenden Tagen beginnend mit dem Tag der Transplantation kommt es auch hier zu einer dosisabhängigen Erhöhung der Überlebenszeiten der Transplantate (siehe Tabelle II).

Tabelle I:

| Wirkung von Verbindung der Formel I mit $R^1$ = H auf die Rattenschwanzhaut-Transplantation bei Fischer-Ratten. | | |
|---|---|---|
| Verbindung (mg/kg; 10xip; d0-9) | Tage bis zur Abstoßung | (x ± s) |
| Kontrolle | 16, 13, 19, 14, 17 | (15.8 ± 2.4) |
| 1 | 26, 22, 21, 24, 21 | (22.8 ± 2.2) |
| 5 | 28, 27, 23, 23, 25 | (25.0 ± 2.6) |
| 10 | 28, 28, 26, 27, 25 | (26.8 ± 1.3) |
| 20 | 26, 27, 26, 24, 23 | (25.2 ± 1.6) |
| 40 | 21, 25, 21, 20, 25 | (22.4 ± 2.4) |
| Verbindung (mg/kg; 10xpo; d0-9) | Tage bis zur Abstoßung | (x ± s) |
| Kontrolle | 20, 16, 19, 18, 19 | (18.4 ± 1.5) |
| 1 | 23, 23, 21, 21, 20 | (21.6 ± 1.3) |
| 5 | 26, 26, 25, 24, 25 | (25.2 ± 0.8) |
| 10 | 28, 28, 27, 29, 30 | (28.4 ± 1.1) |
| 20 | 27, 27, 28, 26, 26 | (26.8 ± 0.8) |
| 40 | 26, 26, 26, 22, 23 | (24.6 ± 2.0) |

Tabelle II:

| Einfluß von Verbindung der Formel I mit $R^1$ = H auf die Hauttransplantation bei Lewis Ratten | | |
|---|---|---|
| Verbindung | Tage bis zur Abstoßung | (x ± s) |
| (mg/kg; 5xip; d0-9) | | |
| Kontrolle | 11, 12, 13, 12, 14 | (12.4 ± 1.1) |
| 5 | 17, 16, 14, 17, 16 | (16.0 ± 1.2)* |
| 10 | 15, 19, 18, 16, 15 | (16.6 ± 1.8)* |
| 25 | 17, 17, 18, 19, 18 | (17.8 ± 0.8)* |
| 50 | 22, 21, 18, 19, 18 | (19.6 ± 1.8)* |
| Verbindung | Tage bis zur Abstoßung | (x ± s) |
| (mg/kg; 10xpo; d0-9) | | |
| 10 | 16, 17, 17, 17, 16 | (16.6 ± 0.6)* |
| 25 | 18, 17, 18, 17, 17 | (17.4 ± 0.6)* |
| 50 | 18, 19, 17, 19, 20 | (18.6 ± 1.1)* |

* p 0.001

Die entsprechend der Erfindung verwendeten Verbindungen sind also in in vivo Testmodellen, die für die Beurteilung von Therapeutika bei Transplantationen herangezogen werden können, imstande, eine Verlängerung der Überlebenszeit der transplantierten Haut zu bewirken. Homopiperazine können also als Therapeutika zur Prophylaxe gegen Transplantatabstoßungen angewendet werden.

## Ansprüche

1. Verwendung einer Verbindung der Formel I

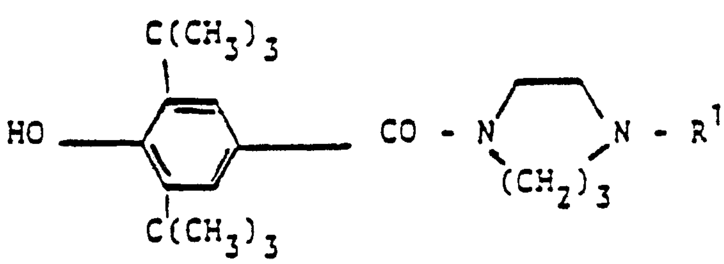

in der $R^1$ ein Wasserstoffatom, eine gradkettige oder verzweigte ($C_1$-$C_6$-Alkoxycarbonyl-gruppe, vorzugsweise $CH_3OCO$ oder tert.-$C_4H_9OCO$, oder die Benzyloxycarbonylgruppe, die im Phenylteil substituiert sein kann, bedeutet, und ihrer physiologisch verträglichen Salze zur Herstellung eines Mittels für die Prophylaxe gegen und Therapie von Organtransplat-Abstoßungsreaktionen.

2. Verwendung nach Anspruch 1, wobei die Verbindung der Formel I in einer Menge von 1-50 mg/kg Körpergewicht verwendet wird.

Patentansprüche für folgende Vertragsstaaten: ES, GR

1. Verfahren zur Herstellung eines Mittels für die Prophylaxe gegen und Therapie von

Organtransplantat-Abstoßungsreaktionen, dadurch gekennzeichnet, daß eine Verbindung der Formel I

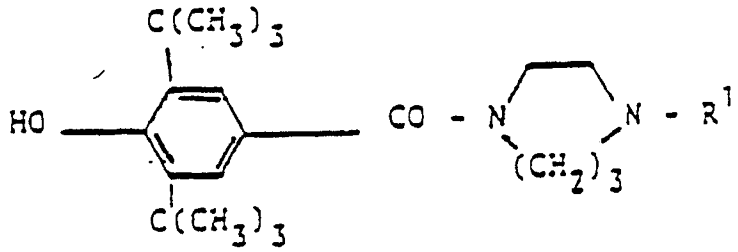

in der $R^1$ ein Wasserstoffatom, eine gradkettige oder verzweigte ($C_1$-$C_6$)-Alkoxycarbonyl-gruppe, vorzugsweise $CH_3OCO$ oder tert.-$C_4H_9OCO$, oder die Benzyloxycarbonylgruppe, die im Phenylteil substituiert sein kann, bedeutet, oder eines ihrer physiologisch verträglichen Salze gegebenenfalls unter Zusatz von Hilfs- oder Zusatzstoffen in eine für eine Anwendung am Menschen geeignete Zubereitung gebracht wird.

2. Verfahren nach Anspruch 1, wobei die Verbindung der Formel I in einer Menge von 75 - 3750 mg pro Dosis (für 75 kg Körpergewicht) in dem hergestellten Mittel enthalten ist.